# EUROPEAN PATENT SPECIFICATION

(11) **EP 0 690 848 B1**
(45) Date of publication and mention of the grant of the patent: **21.01.1998**
(21) Application number: 94912203.0
(22) Date of filing: 15.03.1994
(51) Int. Cl.: C07D 213/74

(54) **PIPERAZINYLPYRIDINYL WATER CLATHRATES**
WASSERCHATHRATE VON PIPERAZINYLPYRIDIN
CLATHRATES AQUEUX DE PIPERAZINYLPYRIDINYLE

(30) Priority: 26.03.1993 US 40181
(43) Date of publication of application: 10.01.1996
(60) Divisional application: 96106831.9
(73) Proprietor: PHARMACIA & UPJOHN COMPANY, Kalamazoo, Michigan 49001 (US)
(72) Inventor: PEARLMAN, Bruce, Allen, Kalamazoo, MI 49008 (US); KROOK, Mark, A., Kalamazoo, MI 49002 (US); PERRAULT, William, R., Kalamazoo, MI 49001 (US); DOBROWOLSKI, Paul, J., Kalamazoo, MI 49008 (US)
(74) Representative: Perry, Robert Edward
(86) International application number: US9402548
(87) International publication number: WO9422836

(56) References cited:
- WO-A-88/07527
- WO-A-93/01181

## Description

### Field of the Invention

The invention relates to 3-alkylamino-2-piperazinylpyridines as water clathrates, and their use.

### Background of the Invention

It is known that certain organic and inorganic molecules form "polyhydrates", also known as "water clathrates", which are crystalline solids that consist of a "host lattice" of water molecules surrounding a "guest molecule". In polyhydrates, molecules of water form a lattice with voids which the guest molecules occupy.

In Prog. Inorg. Chem. 8:43 (1967), in an article entitled the "The Clathrate Hydrates", the subject of polyhydrates was reviewed. This review contains a list of all the organic molecules that were known to form water clathrates at that time. The list is mostly of small molecules such as diethylamine, ethylamine and t-butylamine.

3-Alkylamino-2-piperazinylpyridines are useful as intermediates in the preparation of various pharmaceutical agents; see US-A-4996318, US-A-5120843 and US-A-5175281. Such agents include N-ethyl-2-[4-(5-methoxy-1H-indol-2-ylcarbonyl)-1-piperazinyl]-3-pyridineamine and 2-[4-(5-methanesulfoamido1H-indol-2-ylcarbonyl)-1-piperazinyl]-N-(1-methylethyl)-3-pyridineamine, which are useful in treating individuals who are HIV positive; see WO-A-9109849, Examples 16 and 105.

WO-A-8807527 describes the preparation and work-up of 3-ethylamino-2-piperazinylpiperidine (see Prep. A-47).

### Summary of the Invention

Novel water clathrates according to this invention are 3-ethylamino-2-piperazinylpiperazine.5H₂O and 3-isopropylamino-2-piperazinylpiperidine.5H₂O.

These water clathrates are useful for purifying the parent compounds. They may be prepared by:
(1) adding impure 3-ethylamino-2-piperazinylpyridine or 3-isopropylamino-2-piperazinylpyridine, or a salt or hydrate thereof, to an aqueous mixture,
(2) keeping the pH greater than 7, and
(3) crystallising the water clathrate from the mixture.

### Description of the Invention

3-Alkylamino-2-piperazinylpyridines are produced from the corresponding 3-aminopyridine and piperazine, as described in EP-A-0729948. The starting materials are known.

3-Alkylamino-2-piperazinylpyridines in impure form can be purified by crystallisation from water or an aqueous mixture. The aqueous mixture can contain a water-miscible organic solvent or inorganic or organic salt. The salt is generated upon neutralisation of the reaction mixture.

The compounds of this invention, as obtained by crystallisation from an aqueous medium, are true water clathrates, and not simply mixtures of anhydrous crystals with water, as evidenced by X-ray crystallography.

The following Examples 1 to 4 and 8 illustrate the invention. Examples 5 to 7 are comparative.

All temperatures are in degrees Centigrade.

TLC refers to thin-layer chromatography.

HPLC refers to high-pressure liquid chromatography.

LOD refers to loss on drying.

CMR refers to C-13 magnetic resonance spectroscopy, chemical shifts are reported in ppm (δ) downfield from TMS.

NMR refers to nuclear (proton) magnetic resonance spectroscopy, chemical shifts are reported in ppm (δ) downfield from tetramethylsilane.

MS refers to mass spectrometry expressed as m/e or mass/charge unit. [P + H]⁺ refers to the positive ion of a parent plus a hydrogen atom. CI refers to chemical ionisation.

When solvent pairs are used, the ratios of solvents used are volume/volume (v/v).

When the solubility of a solid in a solvent is used, the ratio of the solid to the solvent is weight/volume (w/v).

### EXAMPLE 1 3-Ethylamino-2-(1-piperazinyl)pyridine pentahydrate

Piperazine (70.10 g), sodium carbonate (22.55 g) and 2-chloro-3-ethylaminopyridine (PREPARATION 1 of EP-A-0729948) are mixed, a condenser with 1 atmosphere steam on it is added and the solid mixture warmed to ≈100° without agitation. The mixture is further heated to reflux at 144°. During the warmup, a large amount of piperazine sublimes into the upper part of the flask, but on reaching reflux this is washed back down. The mixture is stirred at reflux until complete (<1% 2-chloro-3-ethylaminopyridine remains) by HPLC (16 hrs) during which time the reflux temperature slowly rises to 152°. The reaction mixture is then cooled to 147° and toluene (250 ml) is added adiabatically and dropwise. The addition is completed at 74°. The slurry is further cooled to -3°. The solids are collected by vacuum filtration and washed with 0° toluene (2 x 50 ml). The combined filtrate and washes mixed with a toluene rinse. Water (150 ml) is added with stirring. The pH is adjusted from 10.9 to 5.4 with 37 wt% hydrochloric acid (25.17 g) to give a biphasal mixture; the upper phase is discarded. The aqueous phase is adjusted to pH 8.9 with 50 wt% aqueous sodium hydroxide (10.22 g). The mixture is cooled to 20° and seeded with a prior lot of 3-ethylamino-2-(1-piperazinyl)pyridine pentahydrate. The pH is further adjusted to > 12.5 with 50 wt% aqueous sodium hydroxide (15.49 g) while maintaining < 30°. The resultant slurry is cooled to 16°. The product is collected by vacuum filtration and slurry washed with 15° water (50 ml). then displacement washed with 10° water (50 ml). The solids are dried under 34.5 kPa (5 psi) nitrogen for 20 min to give the title compound, TLC eluant: 89/10/1 methylene chloride/methano/29% aq. ammonia, R_{f} = .37; CMR (CDCl₃, ppm δ) 14.80, 38.13, 46.55, 50.56, 115.94, 119.87, 135.21, 137.57, 151.00; NMR (CDCl₃, ppm δ) 1.31, 1.79, 3.02, 3.12, 4.23, 6.81, 6.91, 7.71; MS (CI) m/e = 207 (100.%, P+1), LOD = 30.5%, yield = 80.1% (chemical) from (I).

### EXAMPLE 2 3-Ethylamino-2-(1-piperazinyl)pyridine pentahydrate

Following the general procedure of EXAMPLE 1 and making non-critical variations but not using any base, but only piperazine and heating at reflux (152-158°) for 14 hours, the title compound is obtained, 44.60 g, LOD 46.9%, 73.8% overall yield.

### EXAMPLE 3 A Crystalline Hydrate Of 3-Isopropylamino-2-(1-piperazinyl)pyridine pentahydrate

A small amount of the title compound as a slurry in water is prepared by starting with a small amount of an oily mixture of anhydrous 3-isopropylamino-2-(1-piperazinyl)pyridine (EXAMPLE 3 of EP-A-0729948, ~92% and toluene ~8%] is concentrated under reduced pressure to a viscous oil. A small amount of water is added and the biphasal liquid mixture is frozen at - 25°. On warming to 20-25° and melting, a slurry of the title compound is formed. Crystals from this slurry were used to seed larger lots, which were prepared using the following method.

A liquid mixture of anhydrous 3-isopropylamino-2-(1-piperazinyl)-pyridine (EXAMPLE 3 of EP-A-0729948, ~70% and toluene ~30%, 10.028 g] is concentrated under reduced pressure to a viscous oil (7.106 g). A portion (5.580 g) is mixed with water (30 ml) to give a liquid biphasal mixture. A seed crystal of the title compound is added and on cooling to 0° with stirring, a thick slurry is formed. The product is collected by vacuum filtration and washed with water at 0°. The mixture is dried in an ambient air stream to a workable solid to give the title compound, Karl Fischer = 49.6 wt% water, mp = 26-27°).

EXAMPLEs 4-7 demonstrate that crystallization of the pentahydrate derivative of 3-ethylamino-2-(1-piperazinyl)pyridine (EXAMPLE 4) upgrades the purity much more than does crystallization of either the anhydrous free base (EXAMPLE 6) or the dihydrochloride (EXAMPLE 5).

### EXAMPLE 4 Purification Of 3-Ethylamino-2-(1-piperazinyl)pyridine pentahydrate

To pure 3-ethylamino-2-(1-piperazinyl)pyridine pentahydrate (9.916 g, LOD = 46.4%, 25.765 mmoles) is added pure anhydrous 3-amino-2-(1-piperazinyl)pyridine (62 mg) equivalent to 1.15 wt% on an anhydrous, free base basis. Next, water (25 ml) is added and the mixture is warmed to 48° at which point it becomes an homogeneous solution. The mixture is cooled to 20° and the product collected by vacuum filtration and washed with water (40 ml). The mixture is dried in an air stream for 10 minutes to give 3-ethylamino-2-(1-piperazinyl)-pyridine pentahydrate (7.899 g; LOD = 41.6%; 22.381 mmoles, 86.9%). The product assays by HPLC at 0.1 wt% 3-amino-2-(1-piperazinyl)pyridine on an anhydrous free base basis, reflecting a significant improvement in purity in this crystallization.

### EXAMPLE 5 Purification Of 3-Ethylamino-2-(1-piperazinyl)pyridine dihydrochloride

3-Ethylamino-2-(1-piperazinyl)pyridine dihydrochloride (7.587 g) containing 0.3% of 3-amino-2-(1-piperazinyl)pyridine dihydrochloride is added 3-amino-2-(1-piperazinyl)pyridine dihydrochloride (0.069 g) and water (28 ml). To the resultant solution at pH = 1.5 is added sodium hydroxide (50% aqueous, 4.1 g) to a pH of 9.5. The mixture is seeded with 3-ethylamino-2-(1-piperazinyl)pyridine pentahydrate (III) and then the pH is further adjusted to 12.7 with 3.8 g of 50% aqueous sodium hydroxide. The product is collected under reduced pressure at 20-25° and washed with water (20 ml). The product is dried in a slow air stream for 1 day to give the title compound which assays at 27.0 % water by LOD, mp = 46-48°, and contains no detectable 3-amino-2-(1-piperazinyl)pyridine by TLC.

### EXAMPLE 6 Attempted Purification Of 3-ethylamino-2-(1-piperazinyl)pyridine As Anhydrous Free Base

To pure 3-ethylamino-2-(1-piperazinyl)pyridine pentahydrate (10.268 g, LOD = 46.4%) is added methylene chloride (50 ml). The mixture is warmed to dissolve the solids and the phases separated. The aqueous phase is washed with methylene chloride (10 ml) and the organic phases are combined. To the organic phase is then added pure anhydrous 3-amino-2-(1-piperazinyl)pyridine (64 mg) equivalent to 1.15 wt% on an anhydrous, free base basis. Methyl t-buryl ether (21 ml) and hexane (15 ml) are added and the resultant mixture concentrated under reduced pressure to a total volume of 10 ml. Methyl t-butyl ether (10 ml) is then added and the product is allowed to crystallize at 20-25°. Next, hexane (20 ml) is slowly added to the resultant slurry. The mixture is cooled to 0° and the product collected by vacuum filtration and washed with hexane (~10 ml). The product is dried in a 20-25° nitrogen stream to afford anhydrous 3-ethylamino-2-(1-piperazinyl)pyridine (3.372 g). The product assays by HPLC at 1.5 wt% 3-amino-2-(1-piperazinyl)pyridine, reflecting a decrease in purity in this low yielding crystallization.

### EXAMPLE 7 Attempted Purification Of 3-ethylamino-2-(1-piperazinyl)pyridine dihydrochloride

To pure 3-ethylamino-2-(1-piperazinyl)pyridine pentahydrate (9.988 g, LOD = 46.4%, 25.954 mmoles) is added methylene chloride (50 ml). The mixture is warmed to dissolve the solids and the phases separated. The aqueous phase is washed with methylene chloride (10 ml then 5 ml) and the organic phases combined. To the organic phase is then added pure, anhydrous 3-amino-2-(1-piperazinyl)pyridine (61.0 mg) equivalent to 1.13 wt% on an anhydrous, free base basis. A solution of hydrogen chloride (4.13 g) in methanol (49 ml) is then added with stirring to give an homogeneous solution. Solvent is then distilled off in vacuo to a total volume of about 12 ml. Ethyl acetate (73 ml) is then added and solvent distilled off under reduced pressure to a total volume of about 12 ml. Ethyl acetate (73 ml) is then added and solvent distilled off under reduced pressure to a total volume of about 22 ml. The product is collected by vacuum filtration, washed with ethyl acetate (37 ml), and dried in a 50° vacuum oven for 3 days to give 3-ethylamino-2-(1-piperazinyl)pyridine dihydrochloride (7.215 g, 25.841 mmoles, 99.6% yield). The product assays by HPLC at 1.03 wt% 3-amino-2-(1-piperazinyl)-pyridine on an anhydrous, free base basis. However, only 2.16 mg of 3-amino-2-(1-piperazinyl)pyridine dihydrochloride and 1.39 mg of 3-ethylamino-2-(1-piperazinyl)pyridine dihydrochloride is detected in the filtrate by HPLC, clearly indicating that nearly all the 3-amino-2-(1-piperazinyl)pyridine and 3-ethylamino-2-(1-piperazinyl)pyridine are precipitated as the dihydrochloride salts resulting in insignificant upgrading.

### EXAMPLE8 3-Ethylamino-2-(1-piperazinyl)pyridine pentahydrate

Piperazine (68.20 g, 0.7917 moles), hydrochloric acid (5.16 g, 0.1415 moles) and 2-chloro-3-ethylaminopyridine (26.65 g, containing 7.23% toluene, 24.72 g, 0.1579 moles) are mixed. The solid mixture is warmed to about 110° without agitation. The mixture is further heated to reflux at 152°. The mixture is stirred at reflux until the reaction is complete (<1% 2-chloro-3-ethylaminopyridine) by HPLC (8 hrs). The reflux temperature slowly rises to 168° through the course of the reaction. The mixture is slowly cooled to 126° at which point the piperazine hydrochloride precipitates. Toluene (150 ml) is then added adiabatically and dropwise with good agitation. The addition is completed at 75°. The slurry is further cooled to 3°. The solids are collected by vacuum filtration, washed with 0° toluene (2 x 40 ml) and discarded. The combined filtrate and washes are transferred to a 500 ml flask with a toluene rinse. Water (89 ml) is added with stirring. The pH is adjusted from 11.1 to 4.9 with 20° baume hydrochloric acid (28.41 g, 0.2494 mol. 1.58 eq) to give a liquid biphasal mixture. The upper phase is discarded. The lower phase is washed with toluene (100 ml). The aqueous phase is adjusted to pH 9.1 with aqueous sodium hydroxide (50%, 13.30 g, 0.1663 mol, 1.05 eq). The mixture is cooled to 25° and seeded with a prior lot of the title compound. The pH is further adjusted to > 12.5 with aqueous sodium hydroxide (50%, 11.52 g, 0.1440 mol, 0.91 eq), maintaining < 30°. The resultant slurry is cooled to 12°. The product is collected by vacuum filtration and the slurry washed with 10° water (89 ml), then displacement washed with 10° water (89 ml). The solids are dried under 34.5 kPa (5 psi) nitrogen for 30 minutes to give 3-ethylamino-2-(1-piperazinyl)pyridine pentahydrate (44.60 g, LOD = 34.7%).

## Claims

1. The water clathrate, 3-ethylamino-2-piperazinylpyridine.5H₂O.

2. The water clathrate, 3-isopropylamino-2-piperazinylpyridine.5H₂O.

3. A process for preparing a water clathrate of 3-ethylamino-2-piperazinylpyridine or 3-isopropylamino-2-piperazinylpyridine, which comprises:
(1) adding impure 3-ethylamino-2-piperazinylpyridine or 3-isopropylamino-2-piperazinylpyridine, or a salt or hydrate thereof, to an aqueous mixture,
(2) keeping the pH greater than 7, and
(3) crystallising the water clathrate from the mixture.

4. A process according to claim 3, where the mixture from which the crystallisation takes place contains a salt.

5. A process according to claim 4, where the salt is a chloride, sulfate, bromide, phosphate, hydroxide or carbonate of sodium, potassium, lithium or magnesium.

6. A process according to claim 5, where the salt is sodium chloride or sodium sulfate.

## Patentansprüche

1. Das Wasserclathrat 3-Ethylamino-2-piperazinylpyridin· 5H₂O.

2. Das Wasserclathrat 3-Isopropylamino-2-piperazinylpyridin·5H₂O.

3. Verfahren zur Herstellung eines Wasserclathrats von 3-Ethylamino-2-piperazinylpyridin oder 3-Isopropylamino-2-piperazinylpyridin durch
(1) Eintragen von unreinem 3-Ethylamino-2-piperazinylpyridin oder 3-Isopropylamino-2-piperazinylpyridin oder eines Salzes oder Hydrats derselben in ein wäßriges Gemisch;
(2) Aufrechterhalten eines pH-Werts über 7 und
(3) Kristallisieren des Wasserclathrats aus dem Gemisch.

4. Verfahren nach Anspruch 3, wobei das Gemisch, aus dem die Kristallisation stattfindet, ein Salz enthält.

5. Verfahren nach Anspruch 4, wobei es sich bei dem Salz um ein Chlorid, Sulfat, Bromid, Phosphat, Hydroxid oder Carbonat von Natrium, Kalium, Lithium oder Magnesium handelt.

6. Verfahren nach Anspruch 5, wobei es sich bei dem Salz um Natriumchlorid oder Natriumsulfat handelt.

## Revendications

1. Clathrate aqueux consistant en 3-éthylamino-2-pipérazinylpyridine.5H₂O.

2. Clathrate aqueux consistant en 3-isopropylamino-2-pipérazinylpyridine.5H₂O.

3. Procédé pour la préparation d'un clathrate aqueux de 3-éthylamino-2-pipérazinylpyridine ou de 3-isopropanylamino-2-pipérazinylpyridine, qui comprend :
(1) l'addition de 3-éthylamino-2-pipérazinylpyridine ou de 3-isopropylamino-2-pipérazinylpyridine impure, ou d'un de ses sels ou hydrates, à un mélange aqueux,
(2) le maintien du pH à une valeur supérieure à 7, et
(3) la cristallisation du clathrate aqueux à partir du mélange.

4. Procédé suivant la revendication 3, dans lequel le mélange à partir duquel la cristallisation s'effectue contient un sel.

5. Procédé suivant la revendication 4, dans lequel le sel consiste en un chlorure, sulfate, bromure, phosphate, hydroxyle ou carbonate de sodium, potassium, lithium ou magnésium.

6. Procédé suivant la revendication 5, dans lequel le sel consiste en chlorure de sodium ou sulfate de sodium.
